# EUROPEAN PATENT APPLICATION

(11) **EP 1 403 642 A1**
(43) Date of publication of application: **31.03.2004**
(21) Application number: 02724784.0
(22) Date of filing: 14.05.2002
(51) Int. Cl.: G01N 33/551, G01N 33/553

(54) **PROCESS FOR PRODUCING SUPPORT CARRYING PHYSIOLOGICALLY ACTIVE SUBSTANCE IMMOBILIZED THEREON AND PROCESS FOR PRODUCING THE SAME, IMMOBILIZED PHYSIOLOGICALLY ACTIVE SUBSTANCE, METHOD OF ANALYZING COMPONENT IN SAMPLE AND KIT FOR ANALYZING COMPONENT IN SAMPLE**

(30) Priority: 24.05.2001 JP 2001156086
(71) Applicant: Toyo Kohan Co., Ltd., Tokyo 102-8447 (JP)
(72) Inventor: TANGA, Michifumi, c/o TOYO KOHAN CO.,LTD., Kudamatsu-shi, Yamaguchi 744-8611 (JP); OKAMURA, Hiroshi, c/o TOYO KOHAN CO.,LTD., Kudamatsu-shi, Yamaguchi 744-8611 (JP); TAKAGI, Kenichi, c/o TOYO KOHAN CO.,LTD, Kudamatsu-shi, Yamaguchi 744-8611 (JP); AMANO, Makoto, c/o WAKO PURE CHEMICAL IND., Amagasaki-shi, Hyogo 661-0963 (JP); KUROSAWA, Tatsuo, c/o WAKO PURE CHEMICAL IND. LTD, Amagasaki-shi, Hyogo 661-0963 (JP)
(74) Representative: VOSSIUS & PARTNER
(86) International application number: PCT/JP2002/004661
(87) International publication number: WO 2002/095408

(57) **Abstract**

It is intended to provide a support showing a high immobilization efficiency on which a physiologically active substance can be immobilized at a high density ; a method of efficiently analyzing a biological component by using the immobilization support; and a kit for the analysis. A support for immobilizing a physiologically active substance which comprises a support having a carbon, metallic or a semi-metallic carbon compound layer carrying the physiologically active substance formed on the surface thereof; a process for producing the support for immobilizing a physiologically active substance characterized by bringing a support having a carbon, metallic or a semi-metallic carbon compound layer having functional group into contact with the physiologically active substance; a method of analyzing a component in a sample by using the support : and a kit for analyzing a component in a sample which contains the above support.

## Description

### TECHNICAL FIELD

The present invention provides a support with a physiologically-active substance immobilized thereon, a method for producing the support, a physiologically-active substance immobilized on a support, a method of using the support for analyzing a component in a sample, and a kit with the support for analyzing a component in a sample.

The physiologically-active substance as referred to herein indicates a substance that has a specific action on organisms, or a biological protein or peptide, etc.

### BACKGROUND ART

Most physiologically-active substances such as enzymes and antibodies have high selectivity to specific substances, and, based on such their property, they are utilized for accurate detection of specific biological components. In particular, immunoassay is much used in immunological examinations, and it comprises immobilizing an antibody that reacts with an intended substance on a suitable solid phase (support), then bringing it into contact with a specimen and reacting it with a labeled antibody that may specifically binds to the intended substance.

However, it is extremely difficult to firmly and efficiently immobilize a physiologically-active substance such as protein or saccharide on a support since protein and saccharide have different surface charges.

Heretofore, a method of immobilizing a physiologically-active substance such as protein on a support such as glass slide or polyacrylamide gel, and a method of using the immobilized support for binding an antigen in a sample to the antibody on the support to thereby detect the antigen at a high throughput are reported in *Biotechniques*, 27, 778-788 (1999); *Biosensors & Bioelectronics,* Vol. 13, Nos. 3-4, pp. 407-415, 1998; *Clin*. *Chem.,* 44:9, 2036-2043 (1998); *Clin*. *Chem. Acta*, (1990) 194 (1); *Anal. Chem.,* 1999, 71, 3845-3852; *J. Immunol*. *Methods*, 136 (1991), 239-246; *Anal. Biochem*., 278, 123-131 (2000); *J. Immunol. Methods,* 99 (1987) 107-112; *Anal. Biochem*., 250, 203-211 (1997); *Science,* Vol. 289, 1760-1763, etc.

According to the methods in these reports, however, it is impossible to immobilize an antibody on a support at a high density, and the methods are problematic in that the immobilization efficiency therein is low although they require a large amount of antibody.

For identifying a cause of allergy (allergen), practically employed is a method of detecting IgE. This utilizes the production of IgE that specifically binds to the cause of allergy, allergen through expression of allergy. Specifically, the serum IgE of a case is reacted with an allergen and it is detected with a labeled anti-IgE antibody.

Concretely known are a method that comprises reacting IgE in a sample with an anti-human IgE antibody immobilized on a glass slide, then reacting it with an enzyme-labeled anti-human IgE antibody to produce an anti-human IgE antibody-IgE-enzyme-labeled anti-human IgE antibody complex, and detecting the label in the complex (*Methods in Enzymology,* Vol. 184, 501-507, 1990); a method that comprises binding an allergen to filter paper, reacting it with a serum sample, and detecting it with a *Cypridina*-derived luciferase-labeled anti-IgE antibody (JP-A 5-113443); a method of using an affinity column (JP-T 5-508220 - the term "JP-T" as used herein means a published Japanese translation of a PCT patent application); a method of immobilizing an anti-IgE antibody on a solid phase support such as polystyrene plate or glass fiber filter with a difunctional reagent (JP-T 8-509064), etc.

Even in these methods of these reports, however, it is still impossible to immobilize various allergens or anti-IgE antibodies on a support at a high density, and the methods are still problematic in that the immobilization efficiency therein is low although they require a large amount of antibody.

The invention has been made in consideration of the above-mentioned situation, and its object is to provide a physiologically-active substance-immobilized support on which the density of the physiologically-active substance immobilized is high and the immobilization efficiency is high, a method for producing the immobilized support, a method of using the immobilized support for efficiently analyzing a component in a sample, and a kit for the analysis.

### DISCLOSURE OF THE INVENTION

We, the present inventors have assiduously studied so as to attain the above object and, as a result, have found that, when a support with a carbon or metallic or semi-metallic carbon compound layer formed on its surface is used for immobilizing a physiologically-active substance thereon, then it makes it possible to coordinate a functional group on the support at a high density and therefore a functional group of a physiologically-active substance can be immobilized on the support by binding it to the functional group of the support, and, as a result, the immobilization efficiency is increased and the efficiency in analyzing a component in a sample by the use of the physiologically-active substance-immobilized support is thereby increased. On the basis of this finding, we have reached the present invention.

Specifically, the invention of claim 1 is to provide a physiologically-active substance-immobilized support which has a carbon or metallic or semi-metallic carbon compound layer formed on its surface and has a physiologically-active substance immobilized on the layer.

For immobilizing a physiologically-active substance on the surface of the support, employable is any per-se known immobilization method of, for example, physical adsorbing immobilization or chemical bonding immobilization. Preferably, the substance is immobilized on the support surface via the functional group of the carbon or metallic or semi-metallic carbon compound layer, as in claim 2. More preferably, it is immobilized on the support surface through binding of the functional group of the physiologically-active substance to the functional group of the carbon or metallic or semi-metallic carbon compound layer, as in claim 3.

In this case, it is preferable that the functional group of the physiologically-active substance and/or the functional group of the carbon or metallic or semi-metallic carbon compound layer is one or more selected from a group consisting of a hydroxyl group, a carboxyl group, a sulfo group, a cyano group, a nitro group, a thiol group, an amino group, an aminophenyl group and an epoxy group, as in claim 4.

More preferably, the physiologically-active substance is a substance that has a specific action on organisms or a biological protein or peptide as in claim 5, even more preferably an antigen and/or an antibody as in claim 6. Above all, the physiologically-active substance is especially preferably one or more selected from a group consisting of tumor markers, hormones, hormone-disturbing chemicals, microorganisms-derived proteins or peptides or sugar chain antigens, receptors, ligands, allergens, immunoglobulins, lectins, sugar chains, lipids, lipopolysaccharides and antibodies to these, as in claim 7.

Concretely, the physiologically-active substance is preferably one or more selected from a group consisting of allergens, immunoglobulins and antibodies to these, as in claim 8.

Also preferably, the carbon or metallic or semi-metallic carbon compound layer comprises crystalline carbon and/or amorphous carbon, as in claim 9 and 10. More concretely, the crystalline carbon is preferably diamond or diamond-like carbon as in claim 11, and the amorphous carbon is preferably graphite or amorphous carbon as in claim 12. Of those, more preferred is crystalline carbon, and even more preferred is diamond.

The invention of claim 13 is to provide a method for producing a physiologically-active substance-immobilized support, which comprises contacting a physiologically-active substance with a support having a carbon or metallic or semi-metallic carbon compound layer with a functional formed on its surface.

More concretely, a functional group is introduced into the support surface having a carbon or metallic or semi-metallic carbon compound layer formed thereon, and then this is contacted with a physiologically-active substance, as in claim 14.

In this case, the functional group to be introduced into the support surface is one or more selected from a group consisting of a hydroxyl group, a carboxyl group, a sulfo group, a cyano group, a nitro group, a thiol group, an amino group, an aminophenyl group and an epoxy group, as in claim 15.

The invention of claim 16 is to provide a physiologically-active substance immobilized on a support having a carbon or metallic or semi-metallic carbon compound layer formed thereon.

The invention of claim 17 is to provide a method for analyzing a component in a sample, in which is used the physiologically-active substance-immobilized support of any of claims 1 to 12.

More concretely, the physiologically-active substance-immobilized support is contacted with a sample that contains the component to be analyzed and the resulting complex of the physiologically-active substance and the component is analyzed, as in claim 17.

Preferably, the physiologically-active substance immobilized on the support surface is any of antigen or antibody and the component is an antigen or antibody to the physiologically-active substance, as in claim 19; more preferably, the antigen or antibody is one or more selected from a group consisting of tumor markers, hormones, hormone-disturbing chemicals, microorganisms-derived proteins or peptides or sugar chain antigens, receptors, ligands, allergens, immunoglobulins, lectins, sugar chains, lipids, lipopolysaccharides and antibodies to these, as in claim 20. Above all, the antigen or antibody is more preferably one or more selected from a group consisting of allergens, immunoglobulins, lectins and antibodies to these, as in claim 21.

The invention of claim 22 is to provide a kit for analyzing a component, which contains the physiologically-active substance-immobilized support of any of claims 1 to 11.

### BRIEF DESCRIPTION OF THE DRAWINGS

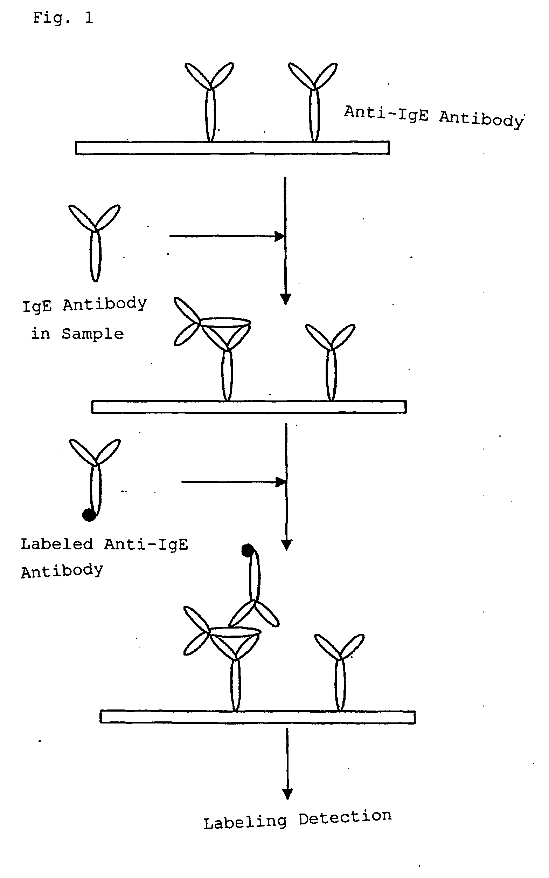

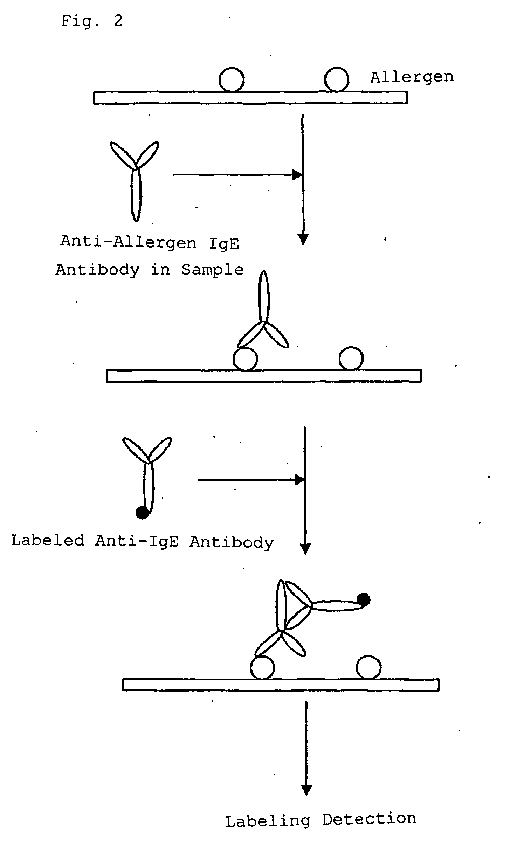

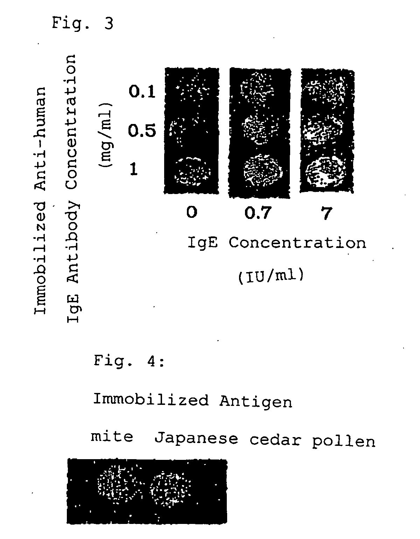

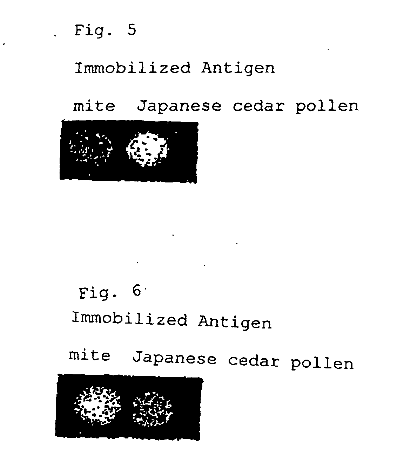

Fig. 1 is a graphical view showing IgE analysis according to the analytical method of the invention. Fig. 2 is a graphical view showing analysis of IgE to a specific allergen according to the analytical method of the invention. Fig. 3 is a view showing the result of IgE analysis in Example 1. Fig. 4 is a view showing the result of allergen analysis in which are used negative serum specimens in Example 2. Fig. 5 is a view showing the result of allergen analysis in which are used Japanese cedar pollen-positive serum specimens in Example 2. Fig. 6 is a view showing the result of allergen analysis in which are used mite-positive serum specimens in Example 2.

### BEST MODES OF CARRYING OUT THE INVENTION

First described is the invention of claim 1, physiologically-active substance-immobilized support.

The physiologically-active substance-immobilized support of claim 1 of the invention is characterized in that it has, on its surface, a carbon or metallic or semi-metallic carbon compound layer with a physiologically-active substance immobilized thereon.

As in claim 5, the physiologically-active substance is a substance having a specific action on organisms, or a biological protein or peptide. Concretely, it includes proteins, protein derivatives, peptides, peptide derivatives, saccharides, saccharide derivatives, lipids, lipid derivatives, etc. More concretely, antigen and/or antibody is preferred as in claim 6, since it enables accurate analysis of biological components through antigen-antibody reaction as will be mentioned hereinunder. Above all, especially preferred are one or more selected from a group consisting of tumor markers (e.g., protein antigens such as AFP, PSA, CEA, PGI, PGH; sugar chain antigens such as CA19-9, PIVKA-11, CA125); hormones (e.g., PTH, T3, T4, TSH, insulin, C-peptide, LH, FSH, prolactin); hormone-disturbing chemicals (e.g., tributyl tin, nonylphenol, 4-octylphenol, di-n-butyl phthalate, dicyclohexyl phthalate, benzophenone, octachlorostyrene, di-2-ethylhexyl phthalate); microorganisms-derived proteins or peptides or sugar chain antigens (e.g., proteins or peptides or sugar chain antigens derived from microorganisms, for example, bacteria such as tubercle bacilli, pneumococci, diphtherococci, meningococci, gonococci, staphylococci, streptococci, enterobacteria, colibacilli, *Helicobacter pylori;* viruses such as influenza viruses, adenoviruses, *Enterovirus,* polioviruses, EB viruses, HAV, HBV, HCV, HIV, HTLV; fungi such as *Candida, Cryptococcus;* leptospire; spirochete such as Treponema pallidum; Chlamydia, Mycoplasma); receptors (e.g., receptors to estrogen, TSH); ligands (e.g., estrogen, TSH); allergens (e.g., aspiratory allergens to cause allergies such as bronchial asthma, allergic rhinitis, atopic dermatitis, for example, food allergens - more concretely, allergens derived from house dusts such as mites, e.g., D. farinae, D. pteronyssinus; pollens of Japanese cedar, cypress, barnyard grass, ragweed, timothy grass, vernal grass, rye grass; animals such as cat, dog, crab; foods such as rice, albumen; and other allergens derived from fungi, insects, woods, drugs and chemical substances, etc.); immunoglobulins (e.g., IgG, IgM, IgA, IgE, IgD, and their degraded products) ; lectins (e.g., concanavalin A, lentil lectin, kidney bean lectin, datura lectin, wheat germ lectin); sugar chains (e.g., ABO antigen); lipids (e.g., lipids such as cholesterol; phospholipids such as cardiolipin, phosphatidylcholine; sphingophospholipids such as sphingomyelin; lipoproteins), lipopolysaccharides (e.g., endotoxin), and antibodies to these, as in claim 7.

In case where any of receptors, ligands, lectins, tumor markers (sugar chains), proteins or peptides is used as the physiologically-active substance, the intended component may be analyzed not only through antigen-antibody reaction but also through receptor-acceptor reaction, lectin-sugar chain reaction or protein-peptide chain reaction.

In case where the physiologically-active substance-immobilized support of the invention is used for analyzing a component in a sample, a suitable physiologically-active substance that specifically binds to the component to be analyzed shall be selected for it. For example, when the component to be analyzed in a sample is an antigen, then an antibody is selected for the physiologically-active substance to be immobilized on a support; and when the component is an antibody, then an antigen to the antibody or an antibody to the antibody is selected.

If two or more different physiologically-active substances are used, then two or more different components in one sample may be simultaneously analyzed in one operation, or unknown components may be analyzed, or detected or specified or identified.

In case where the component to be analyzed is any of receptors, ligands, lectins, tumor markers (sugar chains), proteins or peptides, then a ligand to the receptor, a receptor to the ligand, a sugar chain to the lectin, lectin to the tumor marker (sugar chain), a peptide chain to the protein, or a protein to the peptide, respectively, may be selected for the physiologically-active substance.

In the invention, when a physiologically-active substance-immobilized support that is prepared by immobilizing one or more physiologically-active substances selected from a group consisting of allergens, immunoglobulins (especially IgE) and antibodies to these, on a support is used as in claim 8, then it enables allergy-related analysis, and this is one preferred embodiment of the invention.

Specifically, for example, when an antibody to an allergen is selected for the physiologically-active substance, then it enables allergen analysis (presence or absence of allergen in a sample, and allergen quantification); and when an allergen is selected for the physiologically-active substance, then it enables specific analysis of allergen, a case of allergy (as to the presence or absence of any allergic reaction to what allergen, and as to the degree of the allergic reaction, if any). When an anti-IgE antibody is selected for the physiologically-active substance, then it enables the quantification of the overall IgE amount in a sample (as to whether or not the sample may cause allergic constitution to what degree of allergic reaction).

In case where two or more different physiologically-active substances are immobilized on the surface of a support on which is formed a carbon or metallic or semi-metallic carbon compound layer is formed, as so mentioned hereinabove, it is desirable that the positions at which the physiologically-active substances are immobilized are clarified by suitable expressions that are given to the surface of the support (for example, the positions are numbered).

The amount of the physiologically-active substance to be immobilized on the support that has a carbon or metallic or semi-metallic carbon compound layer formed thereon varies, depending on the surface area of the support and on the property and the type of the physiologically-active substance, and therefore it could not be indiscriminately defined. For example, it may be generally from 0.1 ng to 1 mg, preferably from 1 ng to 100 µg, in terms of the amount of the physiologically-active substance to be immobilized in a unit area (cm²) of the support.

The support of the invention for use in the invention, which as a carbon or metallic or semi-metallic carbon compound layer formed thereon (this may be hereinafter referred to as "carbon or metallic or semi-metallic carbon compound layer-having support) means that the surface of the support is coated with carbon or with a metallic or semi-metallic carbon compound. Concretely, the carbon and the metallic or semi-metallic carbon compound include carbon such as crystalline carbon and amorphous carbon, and a metallic carbon compound and a semi-metallic carbon compound, as in claims 9 and 10.

More concretely, the crystalline carbon is preferably diamond or diamond-like carbon as in claim 11; and the amorphous carbon is preferably graphite or amorphous carbon as in claim 12. The metallic carbon compound is preferably tungsten carbide or titanium carbide; and the semi-metallic carbon compound is preferably silicon carbide. The diamond includes synthetic diamond produced through carbonization, synthetic diamond produced under high pressure, and natural diamond. Regarding its structure, the crystalline carbon compound may have a single-crystal or polycrystal structure.

One or more of these carbons, and metallic or semi-metallic compounds may be used herein either singly or as combined. Preferred are crystalline carbon such as diamond or diamond-like carbon, and amorphous carbon such as graphite or amorphous carbon, and more preferred is crystalline carbon such as diamond or diamond-like carbon, since a large amount of physiologically-active substance may bind to them.

The support to be coated with such a carbon or metallic or semi-metallic carbon compound to give a carbon or metallic or semi-metallic carbon compound layer-having support may be any of inorganic supports, for example, various ceramics such as glass, silica gel, silicon, or alloys of essentially ferrite, iron, nickel or cobalt; or organic supports such as cellulose, dextran, polyacrylamide, polystyrene derivatives, maleic anhydride-based polymers, nylon, polyacrylonitrile and other organic synthetic polymers. The shape of the support is not specifically defined. For example, it may be in any form of granules, plates, rods, membranes, discs or circular discs. Of those, glass is preferred as inexpensive and easily available.

The size of the support varies, depending on the intended use thereof, and is not specifically defined. For example, it may be at most 100 cm², preferably at most 50 cm², more preferably at most 25 cm².

In general, the thickness of the carbon or metallic or semi-metallic carbon compound layer of the carbon or metallic or semi-metallic carbon compound layer-having support is preferably at least 1 nm. More preferably, it is from 1.5 nm to 1000 nm. If the thickness of the layer is smaller than 1 nm, the coating layer will be substantially ineffective; but if larger than 1000 nm, only the extreme surface of the substantial coating layer may be used and it will be useless in point of the labor and the cost in producing the layer.

The carbon or metallic or semi-metallic carbon compound layer may be formed by coating the support with a carbon or metallic or semi-metallic carbon compound in any known method. The known method for it includes microwave plasma CVD, ECRCVD, IPC, DC sputtering, ECR sputtering, ion-plating, arc ion-plating, EB vapor deposition, resistance heating vapor deposition, slurry coating (as in JP-A 10-95695, 8-296044, 8-165576, 8-74056).

The surface of the carbon or metallic or semi-metallic carbon compound layer-having support may be smooth or may be intentionally roughened. Roughening the surface thereof increases the surface area of the support and is therefore advantageous for immobilizing a larger amount of physiologically-active surface thereon.

For immobilizing a physiologically-active substance on the surface of the support, employable is any per-se known immobilization method of, for example, physical adsorbing immobilization or chemical bonding immobilization. Preferably, the substance is immobilized on the support surface via the functional group of the carbon or metallic or semi-metallic carbon compound layer, as in claim 2. More preferably, it is immobilized on the support surface through binding of the functional group of the physiologically-active substance to the functional group of the carbon or metallic or semi-metallic carbon compound layer, as in claim 3.

The functional group of the carbon or metallic or semi-metallic carbon compound layer may be any one capable of binding to a physiologically-active substance either directly or indirectly via a spacer or the like. Concretely, it may be one or more selected from a group consisting of a hydroxyl group, a carboxyl group, a sulfo group, a cyano group, a nitro group, a thiol group, an amino group, an aminophenyl group and an epoxy group, as in claim 4.

Similarly, the functional group of the physiologically-active substance may also be any one capable of binding to the functional group of the carbon or metallic or semi-metallic carbon compound layer, either directly or indirectly via a spacer or the like. For its concretely examples, referred to are those mentioned above for the functional group of the carbon or metallic or semi-metallic carbon compound layer.

Introducing such a functional group into the surface of the carbon or metallic or semi-metallic carbon compound layer-having support may be effected through chemical modification of the support surface to activate it.

For chemical modification of the carbon or metallic or semi-metallic carbon compound layer-having support surface, a functional group such as a hydroxyl group, a carboxyl group, a sulfo group, a cyano group, a nitro group, a thiol group, an amino group, an aminophenyl group or an epoxy group may be directly bound to the support surface. Preferably, however, such a functional group may be bound thereto via a spacer such as a hydrocarbon group having at least 1 carbon atoms, more preferably from 1 to 12 carbon atoms, even more preferably from 1 to 6 carbon atoms. For example, in case where the functional group is a carboxyl group, then a monocarboxylic acid such as formic acid, acetic acid or propionic acid, or a dicarboxylic acid such as oxalic acid, malonic acid, succinic acid, maleic acid or fumaric acid, or a polycarboxylic acid such as trimellitic acid, preferably oxalic acid or succinic acid may be bound to the surface of the carbon or metallic or semi-metallic carbon compound layer.

For introducing such a functional group to the chemically-modified surface of the carbon or metallic or semi-metallic carbon compound layer-forming support, directly or via a spacer, for example, employable is a method of oxidizing the support surface with oxygen plasma followed by processing it with steam; or a method of processing the support surface with steam, then exposing it to UV rays in chlorine gas to thereby chlorinate the support surface, and thereafter hydrolyzing it in an alkali solution to hydroxylate it; or a method of oxidizing the support surface with oxygen plasma, then chlorinating it, and hydrolyzing it in an alkali solution to hydrolyze it; or a method of hydrogenating the support surface, then exposing it to UV rays in chlorine gas to chlorinate it, and thereafter reacting it with a sodium carboxylate in a non-aqueous solvent.

More concretely, for example, when the surface of the carbon or metallic or semi-metallic carbon compound layer-having support is to be chemically modified with a carboxyl group, it is desirable that the surface of the carbon or metallic or semi-metallic carbon compound layer-having support is hydrogenated, chlorinated, aminated and then carboxylated. Precisely, it is desirable that the support surface is hydrogenated, then the surface of the carbon material on the support is chlorinated through exposure to UV rays in chlorine gas, and thereafter it is aminated through exposure to UV rays in ammonia gas and then condensed with a dicarboxylic acid.

Immobilization of a physiologically-active substance on the carbon or metallic or semi-metallic carbon compound layer-having support may be effected in the manner mentioned below, based on the type of the functional group existing in the support surface (for example, as in *Methods in Enzymology,* Vol. 44, pp. 11-148 (1976)).
(1) When the functional group of the carbon or metallic or semi-metallic carbon compound layer-having support is an amino group:
   When the surface of the carbon or metallic or semi-metallic carbon compound layer-having support is chemically modified with an amino group, then the carboxyl group of the physiologically-active substance may be bound to the amino group in the support surface by the use of a condensation reagent such as N-hydroxysuccinimide or carboxydiimide whereby the physiologically-active substance may be immobilized on the support surface.
   Apart from it, the amino group of the support surface may be isocyanated through reaction with phosgene, and thereafter it may be bound to a physiologically-active substance-derived amino group to thereby immobilize the physiologically-active substance on the support surface. Further, the amino group of the support surface may be isothiocyanated through reaction with thiophosgene, and thereafter it may be bound to a physiologically-active substance-derived amino group whereby the physiologically-active substance may also be immobilized on the support surface.
   Still further, the amino group of the support surface may be reacted with glutaraldehyde, and this may be reacted with a physiologically-active substance-derived amino group or phenol group to thereby immobilize the physiologically-active substance on the support surface.
   When the physiologically-active substance is a saccharide or saccharide derivative such as glycoprotein, then it may be first reacted with periodic acid and then with the amino group of the support surface, and thereafter it may reduced with sodium borohydride or the like whereby the substance may be immobilized on the support surface.
   On the other hand, the amino group of the support surface may be maleimidated or pyridyldithiosulfidated with a maleimido group-having difunctional spacer such as m-maleimidobenzoyl N-hydroxysuccinimide ester, or a pyridyldithiosulfido group-having difunctional spacer such as 4-succinimidyloxycarbonyl-α-(2-pyridyldithio)toluene, and the resulting maleimidated or pyridyldithiosulfidated support is reacted with the thiol group of the physiologically-active substance to thereby immobilize the substance on the support surface.
(2) When the functional group of the carbon or metallic or semi-metallic carbon compound layer-having support is a carboxyl group:
   When the surface of the carbon or metallic or semi-metallic carbon compound layer-having support is chemically-modified with a carboxyl group, then the carboxyl group of the support surface may be reacted with the amino group of the physiologically-active substance by the use of a condensation reagent such as N-hydroxysuccinimide or carbodiimide, whereby the physiologically-active substance may be immobilized on the support surface. Apart from it, the carboxyl of the support surface may be reacted with thionyl chloride to form a carboxy chloride. The resulting carboxy chloride may be reacted with a physiologically-active substance-derived amino group to thereby immobilize the substance on the support surface.
   Further, the carboxyl group of the support surface may be reacted with hydrochloric acid and methanol added thereto, and then reacted with hydrazine also added thereto to form a hydrazide, and the resulting hydrazide is acylazidated with sodium nitride and hydrochloric acid added thereto to active the support surface, and thereafter the thus-activated support surface is reacted with a physiologically-active substance-derived amino, thiol or hydroxyl group to immobilize the substance on the support surface.
   Still further, the carboxyl group of the support surface is converted into its anhydride, and this is reacted (through dehydrating condensation) with a physiologically-active substance-derived amino group to thereby immobilize the substance on the support surface.
(3) When the functional group of the carbon or metallic or semi-metallic carbon compound layer-having support is a hydroxyl group:
   When the surface of the carbon or metallic or semi-metallic carbon compound layer-having support is chemically modified with a hydroxyl group, then the hydroxyl group of the coated support surface is reacted with cyanuric chloride to form a triazinyl derivative, to which is bound a physiologically-active substance-derived amino group to thereby immobilize the substance on the support surface.
   Apart from it, when the support surface is chemically modified with a hydroxyl group, then the hydroxyl group of the support surface may be acetylated and the brominated, and this is then reacted with NaI for substitution of bromine therein with iodine to thereby active the support surface, and thereafter this is reacted with a physiologically-active substance-derived amino, thiol or hydroxyl group to immobilize the substance on the support surface.
   Further, the hydroxyl group of the support surface may be activated through reaction with cyanogen bromide, and this may be covalent-bonded to a physiologically-active substance-derived amino group to immobilize the substance on the support surface.
(4) When the functional group of the carbon or metallic or semi-metallic carbon compound layer-having support is an epoxy group:
   When the surface of the carbon or metallic or semi-metallic carbon compound layer-having support is chemically modified with an epoxy group, then it may be reacted with a physiologically-active substance-derived hydroxyl group (such as that derived from tyrosine or serine), or amino or thiol group to thereby immobilize the substance on the support surface.
(5) When the functional group of the carbon or metallic or semi-metallic carbon compound layer-having support is a sulfo group:
   When the surface of the carbon or metallic or semi-metallic carbon compound layer-having support is chemically modified with a sulfo group, then the sulfo group of the support surface may be reacted with chlorosulfonic acid to form a sulfone chloride, and this is then reacted with a physiologically-active substance-derived amino, imidazole, thiol or phenol group to immobilize the substance on the support surface.
(6) When the functional group of the carbon or metallic or semi-metallic carbon compound layer-having support is an aminophenyl group:
   When the surface of the carbon or metallic or semi-metallic carbon compound layer-having support is chemically modified with an aminophenyl group, then the aminophenyl group of the support surface may be activated with sodium sulfite and hydrochloric acid, and this is then reacted with a physiologically-active substance-derived amino, thiol, phenol, imidazole or guanidino group in a mode of diazonium coupling to thereby immobilize the substance on the support surface.
(7) When the functional group of the carbon or metallic or semi-metallic carbon compound layer-having support is a cyano group:
   When the surface of the carbon or metallic or semi-metallic carbon compound layer-having support is chemically modified with a cyano group, then the cyano group may be hydrolyzed in the presence of an acid catalyst and thereafter converted into a carboxyl group. With that, the support may be processed in the same manner as in the above (2) for physiologically-active substance immobilization thereon.
(8) When the functional group of the carbon or metallic or semi-metallic carbon compound layer-having support is a nitro group:
   When the surface of the carbon or metallic or semi-metallic carbon compound layer-having support is chemically modified with a nitro group, then the nitro group may be reduced with a reducing agent such as lithium aluminium hydride and thereafter converted into an amino group. With that, the support may be processed in the same manner as in the above (1) for physiologically-active substance immobilization thereon.
(9) When the functional group of the carbon or metallic or semi-metallic carbon compound layer-having support is a thiol group:
   When the surface of the carbon or metallic or semi-metallic carbon compound layer-having support is chemically modified with a thiol group, then the amino group of a physiologically-active substance may be maleimidated or pyridyldithiosulfidated with a maleimido group-having difunctional spacer such as m-maleimidobenzoyl N-hydroxysuccinimide ester, or a pyridyldithiosulfido group-having difunctional spacer such as 4-succinimidyloxycarbonyl-α-(2-pyridyldithio)toluene, and the resulting maleimidated or pyridyldithiosulfidated, physiologically-active substance is reacted with the thiol group of the support surface to thereby immobilize the substance on the support surface.

Using a different difunctional spacer such as bismaleimidohexane or 1,4-di-[3'-2'-pyridyldithio (propionamido)]butane may be used in place of the above-mentioned one, the thiol group of the support surface may be reacted with the thiol group of a physiologically-active substance to immobilize the substance on the support surface.

The above-mentioned methods may apply to any other cases where the combination of the functional groups to be bound to each other is the same as in those methods, even though the functional group of the support surface and that of the physiologically-active substance are contrary to those in the methods.

Preferably, the physiologically-active substance-immobilized support of the invention that has a physiologically-active substance immobilized on the surface of the carbon or metallic or semi-metallic carbon compound layer formed thereon is blocked with a blocking agent, more concretely with albumin, globulin, casein, polyvinyl alcohol, surfactant, silane coupling agent, titanium coupling agent, aluminium coupling agent or the like, for preventing the influence of any non-specific adsorption thereto on the result of analysis with it. The support of the invention may be stored in different and various conditions, for example, after it is dried, frozen or lyophilized, or while it is in a suitable buffer.

The buffer may be any one generally used in the art, including, for example, tris-buffer, phosphate buffer, Veronal buffer, borate buffer, Good buffer, etc. The buffer of the type may contain any of albumin, globulin, water-soluble gelatin, polyethylene glycol and other stabilizers, surfactants and saccharides.

For producing the physiologically-active substance-immobilized support of the invention where the carbon or metallic or semi-metallic carbon compound layer formed on its surface has two or more different physiologically-active substances immobilized thereon, the same processes as above are employable except that two or more suitable physiologically-active substances are used in producing it.

Next described is the method for producing the physiologically-active substance-immobilized support of the invention where the carbon or metallic or semi-metallic carbon compound layer formed on its surface has a physiologically-active substance immobilized thereon.

As in claim 13, a physiologically-active substance is contacted with a support having a carbon or metallic or semi-metallic carbon compound layer with a functional formed on its surface, and the substance is immobilized on the support surface in any per-se known immobilization method as in the above, for example, in a method of making the support physically adsorb the substance for the substance immobilization thereon (physical binding method). In that manner, the intended, physiologically-active substance-immobilized support is produced, in which the carbon or metallic or semi-metallic carbon compound layer formed on its surface has a physiologically-active substance immobilized thereon.

Preferably, the support having a carbon or metallic or semi-metallic carbon compound layer with a functional group formed on its surface is contacted with a physiologically-active substance to thereby immobilize the substance on the support surface through the chemical bonding of the substance to the functional group of the carbon or metallic or semi-metallic carbon compound layer (chemical bonding method), as it enables firmer binding between the support and the physiologically-active substance. In particular, it is more desirable that the functional group of the physiologically-active substance is chemically bonded to the functional group of the carbon or metallic or semi-metallic carbon compound layer to thereby immobilize the substance on the support surface.

For the production method of the invention, the specific examples and the preferred embodiments of the physiologically-active substance, the carbon or metallic or semi-metallic carbon compound and the support, as well as the method of forming the carbon or metallic or semi-metallic carbon compound layer may be the same as those mentioned hereinabove.

Further, the type and the combination of the functional group to be introduced into the carbon or metallic or semi-metallic carbon compound layer, and the functional group of the physiologically-active substance to be bound to the former may also be the same as those mentioned hereinabove. The functional group of the physiologically-active substance and that of the carbon or metallic or semi-metallic carbon compound layer may be any ones capable of binding to each other either directly or indirectly via a spacer or the like. Concretely, they may be one or more selected from a group consisting of a hydroxyl group, a carboxyl group, a sulfo group, a cyano group, a nitro group, a thiol group, an amino group, an aminophenyl group and an epoxy group, as in claim 15.

Concretely, the production method of the invention may be carried out, for example, as follows:

In the physical binding method, for example, a physiologically-active substance-containing solution is applied to the carbon or metallic or semi-metallic carbon compound layer-having support, for example, in a mode of coating, dropping application or spraying, or the support is dipped in the solution, or a commercially-available stamping device (for example, that from Nippon Laser Electronics) is used to thereby make the support contacted with the physiologically-active substance. Thus processed, the support is dried to thereby make the physiologically-active substance immobilized on its surface through physical adsorption. In that manner, the intended, physiologically-active substance-immobilized support is produced, in which the carbon or metallic or semi-metallic carbon compound layer formed on its surface has a physiologically-active substance immobilized thereon.

In the chemical bonding method, for example, a physiologically-active substance-containing solution is applied to the carbon or metallic or semi-metallic carbon compound layer-having support having a functional group in its surface, for example, in a mode of coating, dropping application or spraying, or the support is dipped in the solution, or a commercially-available stamping device (for example, that from Nippon Laser Electronics) is used to thereby make the support contacted with the physiologically-active substance. With that, the functional group of the support is reacted with the physiologically-active substance, concretely with the functional group of the physiologically-active substance to chemically bond the two through per-se known chemical bonding between the two and to thereby immobilize the physiologically-active substance on the support surface. In that manner, the intended, physiologically-active substance-immobilized support is produced, in which the carbon or metallic or semi-metallic carbon compound layer formed on its surface has a physiologically-active substance immobilized thereon.

In case where the carbon or metallic or semi-metallic carbon compound layer-having support does not have a suitable functional group to which a physiologically-active substance is bound, then a suitable functional group is first introduced into the surface of the carbon or metallic or semi-metallic carbon compound layer-having support, and then the support may be contacted with a physiologically-active substance in the manner as above, as in claim 14.

In the methods mentioned above, the related description give hereinabove may be referred to for the mode of introducing the functional group into the surface of the carbon or metallic or semi-metallic carbon compound layer-having support.

In the methods mentioned above, any one generally used in the art may be used in preparing the physiologically-active substance-containing solution. For example, it includes tris-buffer, phosphate buffer, Veronal buffer, borate buffer and Good buffer. The amount of the physiologically-active substance to be in the solution thereof for immobilization could not be defined indiscriminately, as varying depending on the property and the type of the physiologically-active substance to be used. In general, it may be from 1 ng/ml to 1000 mg/ml, preferably from 1 µg/ml to 10 mg/ml, more preferably from 10 µg/ml to 2 mg/ml. Using the physiologically-active substance-containing solution of the type, the substance is immobilized on the support surface so that its concentration may fall within the range mentioned above.

The invention of claim 16 relates to the physiologically-active substance immobilized on the support that has a carbon or metallic or semi-metallic compound layer formed thereon.

The immobilized physiologically-active substance of the type is heretofore unknown, and it enables accurate and highly-sensitive detection of a component in a sample that has the property of binding to the physiologically-active substance.

For the immobilized physiologically-active substance of the type, the specific examples and the preferred embodiments of the physiologically-active substance, the carbon or metallic or semi-metallic carbon compound and the support, as well as the method of forming the carbon or metallic or semi-metallic carbon compound layer and the immobilization method may be the same as those mentioned hereinabove.

After the support in which the carbon or metallic or semi-metallic carbon compound layer formed on its surface has the physiologically-active substance immobilized thereon is obtained according to the above-mentioned method, it is desirable that the support is blocked in an ordinary manner generally employed in the art, for example, by dipping it in a solution that contains a blocking agent such as albumin, globulin, casein, polyvinyl alcohol, surfactant, silane coupling agent, titanium coupling agent, aluminium coupling agent or the like.

Next described is the method of claim 17 of the invention for analyzing a component in a sample.

In the method of claim 17 of the invention, used is the physiologically-active substance-immobilized support of any of claims 1 to 12. Using the physiologically-active substance-immobilized support enables efficient, highly-sensitive and accurate analysis of a component in a sample.

More concretely, the physiologically-active substance-immobilized support of any of claims 1 to 12 is first contacted with a sample that contains the component to be analyzed to thereby form a complex of the physiologically-active substance immobilized on the support and the component in the sample, as in claim 18. Next, the resulting complex is analyzed to analyze the component in the sample.

When the physiologically-active substance-immobilized support for use in the analytical method of the invention has two or more different physiologically-active substances immobilized on the surface of the carbon or metallic or semi-metallic carbon compound layer formed thereon, then the intended component in a sample may be more efficiently, more sensitively and more accurately analyzed and, in addition, two or more different components in one sample may be simultaneously analyzed in one operation and unknown components may be analyzed (specified, identified).

More concretely, a sample is applied onto the surface of the support in such a manner that all the components in the sample may be contacted substantially all at a time with the two or more different physiologically-active substances immobilized on the support, and the resulting complexes of each immobilized, physiologically-active substance and each component in the sample are analyzed to thereby analyze the intended components in the sample.

The component to be analyzed herein is one having the ability to specifically bind to a physiologically-active substance, for example, through antigen-antibody reaction, receptor-ligand reaction, lectin-sugar chain reaction or protein-peptide chain reaction. More concretely, it includes the above-mentioned physiologically-active substances. For the combination of the physiologically-active substance and the component to be analyzed, referred to are those mentioned hereinabove.

The sample includes, for example, body fluids such as serum, plasma, spinal fluid, synovial fluid, lymphatic fluid; discharges such as urine, feces; other biological samples such as sputum, pus, skin-derived matters; foods, drinks, tap water, seawater, lake water, marsh water, river water, industrial wastes, semiconductor washes and other environmental samples such as washes of medical instruments; and those reconstructed from them by suitably dissolving them in water or in a buffer generally employed in the art, such as tris-buffer, phosphate buffer, Veronal buffer, borate buffer or Good buffer.

For contacting the physiologically-active substance immobilized support in which the carbon or metallic or semi-metallic carbon compound layer formed on its surface has a physiologically-active substance immobilized thereon, with the sample that contains a component to be analyzed, and for contacting all the components to be analyzed in a sample with the physiologically-active substance immobilized support that has two or more different types of physiologically-active substances immobilized thereon, substantially all at a time in the analytical method of the invention, for example, employable is a method of applying the sample to the support in a mode of coating or dropping application, or a method of dipping the support in the sample.

For analyzing the complex of the physiologically-active substance immobilized on the support and the component in the sample in the above-mentioned method, in general, a labeled binding substance that has the property of specifically binding to the component or to the complex is contacted with the complex to thereby form a complex (labeled complex) of the immobilized physiologically-active substance, the component in the sample and the labeled binding substance, and the labeling substance in the labeled complex is detected. On the basis of the thus-detected data, the complex of the physiologically-active substance and the component in the sample is analyzed.

In case where the component to be analyzed is one directly detectable in some method, for example, enzyme, dye, fluorescent substance, luminescent substance, or UV-absorbable substance, it does not always require the above-mentioned labeled binding substance. Contacting the complex of the immobilized physiologically-active substance and the component in the sample with the labeled binding substance that has the property of specifically binding to the complex may be effected in the same manner as that for contacting the physiologically-active substance-immobilized support that has a physiologically-active substance immobilized on the carbon or metallic or semi-metallic carbon compound layer formed therein, with the component-containing sample.

The labeled binding substance that has the property of specifically binding to the component or to the complex is one having the property of specifically binding to the component or the complex and capable of being detected in some method. In general, it is a substance labeled with a labeling substance and having the ability to specifically bind to the component or to the complex. The substance having the ability to specifically bind to the component or to the complex is the same as the physiologically-active substance mentioned hereinabove, and it is generally an antibody or antigen to the component or the complex.

The labeling substance for use in the invention may be any one generally employable in the art for enzyme immunoassay (EIA), radiation immunoassay (RIA), fluorescence immunoassay (FIA), hybridization or the like. For example, it includes enzymes such as alkali phosphatase (ALP), β-galactosidase (β-Gal), peroxidase (POD), microperoxidase, glucose oxidase (GOD), glucose-6-phosphate dehydrogenase (G6PDH), malate dehydrogenase, luciferase; dyes such as Coomassie Brilliant Blue R250, methyl orange; radioactive isotopes such as ^{99m}Tc, ¹³¹I, ¹²⁵I, ¹⁴C, ³H, ³²P, ³⁵S; Cy3, fluorescein, rhodamine, dansyl, fluorescamin, coumarin, naphtylamine and their derivatives; fluorescent substances such as europium (Eu); luminescent substances such as luciferin, isoluminol, luminol, bis(2,4,6-trichlorophenyl) oxalate; UV-absorbable substances such as phenol, naphthol, anthracene and their derivatives; substances having the property as a spin-labeling agent, such as typically oxyl group-having compounds, e.g., 4-amino-2,2,6,6-tetramethylpiperidin-1-oxyl, 3-amino-2,2,5,5-tetramethylpyrrolidin-1-oxyl, 2,6-di-t-butyl-α-(3,5-di-t-butyl-4-oxo-2,5-cyclohexadien-1-ylidene)-p-tolyloxyl.

Labeling the component or the complex with the labeling substance that has the ability to specifically bind to it may be effected in any ordinary method generally employable in the art, for example, according to a per-se known labeling method generally employed in per-se known EIA, RIA, FIA or hybridization [for example, as in *Medicochemical Experiment Lecture,* Vol. 8, consulted by Yuichi Yamamura, 1st Ed., Nakayama Publishing, 1971; *Illustrated Fluorescent Antibody,* written by Akira Kawao, 1st Ed., Softscience, 1983; *Enzyme Immunoassay*, edited by Eiji Ishikawa, Tadashi Kawai, Kiyoshi Miyai, 3rd Ed., Igaku Shoin, 1987; *Molecular Cloning A Laboratory Manual,* 2nd Ed., J. Sambrook, E. F. Frish, T. Maniatis, Cold Spring Harbor Laboratory Press], or in any other ordinary method of utilizing the reaction of avidin (or streptoavidin) and biotin.

In the analytical method of the invention, the presence or absence of the intended component in a sample is confirmed through detection of the component on the basis of the property thereof in the complex formed of the physiologically-active substance-immobilized support, in which the carbon or metallic or semi-metallic carbon compound layer formed on its surface has one or more different physiologically-active substances immobilized thereon, and the component in the sample, or the property of the labeling substance in the complex (labeled complex) formed of the physiologically-active substance-immobilized support, in which the carbon or metallic or semi-metallic carbon compound layer formed on its surface has one or more different physiologically-active substances immobilized thereon, the component in the sample, and a labeled binding substance that has the property of specifically binding to the component or to the complex of the physiologically-active substance and the component.

In addition, in the analytical method of the invention, it is possible to determine the amount of the component in the complex thereof or the amount of the labeling substance in the labeled complex thereof in accordance with the property of component or the labeling substance, and on the basis of the thus-determined data, it is possible to quantitatively or semi-quantitatively determine the amount of the component in the sample.

For computing the amount of the component in the sample on the basis of the determined amount of the component or that of the labeling substance, for example, samples (standard samples) each containing a known concentration of the component are analyzed in the same manner as above to form a calibration curve that indicates the relationship between the component concentration and the measured data (measured data of the component in the complex or those of the labeling substance in the labeled complex), and the amount of the intended component in the sample is determined on the basis of the thus-prepared calibration curve.

The analytical method of the invention is described more concretely.

First, the physiologically-active substance-immobilized support, in which the carbon or metallic or semi-metallic carbon compound layer formed on its surface has one or more different physiologically-active substances immobilized thereon, is contacted with the sample that contains the intended component in the manner as above to thereby form a complex of the physiologically-active substance and the component. Next, the support is washed with a buffer that is used in the filed of hybridization of immunoassay, such as tris-buffer, phosphate buffer, Veronal buffer, borate buffer, Good buffer or SSC buffer to thereby remove the components not having participated in the complex formation in the sample. Next, in short, the thus-formed complex is contacted with a labeled binding substance that has the property of specifically binding to the component or to the complex to thereby form a labeled complex of the immobilized physiologically-active substance, the component and the labeled binding substance, and thereafter the support is washed with a buffer such as that mentioned above to thereby remove the free, labeled binding substance not having participated in the formation of the labeled complex. Next, the component in the complex and the labeling substance in the labeled complex is determined in a predetermined method in accordance with the property thereof, and on the basis of the thus-determined data, the component in the sample may be analyzed.

Apart from the methods mentioned hereinabove, the intended component in a sample may also be analyzed in any other method, for example, through competitive assay in which the component in the sample is competitively reacted with a component of the same type but labeled with a labeling substance.

Concretely, the physiologically-active substance-immobilized support in which the carbon or metallic or semi-metallic carbon compound layer formed on its surface has one or more different physiologically-active substances immobilized thereon is contacted with a sample that contains the intended component and with a component of the same type as that of the intended component but labeled with a labeling substance to thereby form a labeled complex of the physiologically-active substance and the labeled component, and a complex of the physiologically-active substance and the component. Next, the support is washed with a buffer such as that mentioned above to thereby remove the component in the sample and the free labeled component not having participated in the complex formation. Next, the labeling substance in the labeled complex or the labeling substance having bound to the removed, free labeled component is determined in a predetermined method in accordance with the property thereof, and, on the basis of the thus-measured data, the intended component in the sample may be analyzed.

In this embodiment, the method of labeling the component with a labeling substance and the method of contacting the physiologically-active substance-immobilized support with the sample and with the labeled component may be the same as the method of labeling the component or the substance having the ability to specifically bind to the complex, with a labeling substance such as that mentioned above, and the method of contacting the physiologically-active substance-immobilized support with the component-containing sample, respectively, and the others are also the same as those mentioned hereinabove.

In the above-mentioned methods, determination of the component in the complex or the labeling substance in the labeled complex may be effected in a predetermined manner in accordance with the type of the component and the labeling substance. For example, when the property of the substance to be determined is enzymatic activity, then any known method of EIA or hybridization may be employed. For example, the determination may be effected in accordance with the methods described in *Enzyme Immunoassay,* Protein, Nucleic Acid, Enzyme, Separate Edition, No. 31, edited by Tsunehiro Kitagawa, Toshio Minamihara, Akio Tsuji, Eiji Ishikawa, pp. 51-63, published by Kyoritsu Publishing, September 10, 1987. When the substance to be detected is a radioactive substance, then it may be determined in any ordinary method of RIA or hybridization. Concretely, a suitable instrument such as dip GM counter, liquid scintillation counter or square-well scintillation counter is selected and used in accordance with the type and the intensity of the radiation to be emitted by the radioactive substance and the substance is thereby detected and determined (for example, see as in *Medicochemical Experiment Lecture,* Vol. 8, consulted by Yuichi Yamamura, 1st Ed., Nakayama Publishing, 1971; *Biochemical Experiment Lecture 2,* Tracer Experiment Method, latter edition, by Shosuke Takemura, You Honsho, pp. 501-525, published by Tokyo Kagaku Doj in, February 25, 1977). When the property of the substance to be determined is fluorescence, then the substance may be determined in any known method of FIA or hybridization that uses a metering instrument of fluorophotometer or confocal laser microscope. Concretely, for example, employable for it are the methods described in *Illustrated Fluorescent Antibody,* written by Akira Kawao, 1st Ed., Softscience, 1983; *Biochemical Experiment Lecture 2,* Chemistry of NucleicAcid III, by Minero Jitsuyoshi, pp. 299-318, published by Tokyo Kagaku Dojin, December 15, 1977. When the property of the substance to be determined is luminescence, then the substance may be determined in any known method of using a metering instrument such as photon counter. Concretely, for example, employable for it are the methods described in *Enzyme Immunoassay,* Protein, Nucleic Acid, Enzyme, Separate Edition, No. 31, edited by Tsunehiro Kitagawa, Toshio Minamihara, Akio Tsuji, Eiji Ishikawa, pp. 252-263, published by Kyoritsu Publishing, September 10, 1987. When the property of the substance to be determined is UV absorption, then the substance may be determined in any known method of using a metering instrument such as spectrophotometer. When the property of the substance to be determined is color expression, then the substance may be determined in any known method of using a metering instrument such as spectrophotometer or microscope. When the property of the substance to be detected is spinning, then an ordinary method of using an electronic spin resonance device may be employed for determining the substance. Concretely, for example, employable for it are the methods described in *Enzyme Immunoassay,* Protein, Nucleic Acid, Enzyme, Separate Edition, No. 31, edited by Tsunehiro Kitagawa, Toshio Minamihara, Akio Tsuji, Eiji Ishikawa, pp. 264-271, published by Kyoritsu Publishing, September 10, 1987.

In the above-mentioned methods, the amount of the labeled binding substance that has the property of specifically binding to the component to be analyzed or to the complex of the component varies, depending on the type of the labeled binding substance used, and therefore could not be indiscriminately defined. In general, however, the amount is not lower than the concentration of the substance capable of binding to all the physiologically-active substances immobilized on the support. Similarly, the amount of the component labeled with a labeling substance could not be indiscriminately defined, as varying depending on the type of the component to be analyzed. In general, however, it is not lower than the concentration of the component capable of binding to all the physiologically-active substances immobilized on the support.

In the above-mentioned methods, the pH and the temperature during the reaction could not also be indiscriminately defined, as varying depending on the type of the physiologically-active substance used and the component to be analyzed. They may be within the range within which they do not interfere with the formation of the complex of the physiologically-active substance and the component, or the labeled complex of the physiologically-active substance, the component and the labeling substance, or the complex of the physiologically-active substance and the labeled component. Concretely, the pH falls generally between 2 and 10, preferably between 5 and 9; and the temperature falls generally between 0 and 90°C, preferably between 20 and 80°C. The reaction time may be suitably determined in accordance with the properties of the components to form the complexes, since the time necessary for forming the complexes as above varies depending on the properties of the constitutive components. In general, the components may be suitably reacted for a few seconds to a few hours.

In the analytical methods of the invention, when an antigen or antibody is selected for the physiologically-active substance to be immobilized on the support surface as in claim 19, then the antigen or antibody to the physiologically-active substance in a sample may be accurately analyzed in a simplified manner. When one or more are selected from a group consisting of tumor markers, hormones, hormone-disturbing chemicals, microorganisms-derived proteins or peptides or sugar chain antigens, receptors, ligands, allergens, immunoglobulins (especially IgE), lectins, sugar chains and antibodies to these as in claim 20, then these themselves or the antibody to these physiologically-active substances in a sample may be analyzed in a simplified manner, and this is a favorable embodiment of the invention.

When receptors, ligands, lectins, tumor markers (sugar chains), proteins or peptides are selected for the physiologically-active substance, then they may be analyzed not only through antigen-antibody reaction but also through receptor-ligand reaction, lection-sugar chain reaction or protein-peptide chain reaction.

Further, when the analytical methods of the invention are carried out by the use of the physiologically-active substance-immobilized support of the invention in which the physiologically-active substance is one or more selected from a group consisting of allergens, immunoglobulins (especially IgE) and antibodies to these as in claim 21, then allergy-related analysis may be effected accurately and in a simplified manner, and this embodiment is especially favorable in the invention.

Concretely, for example, when an antibody to allergen is used for the physiologically-active substance, then it is favorable for allergen analysis (in point of the presence or absence of allergen in a sample and of the amount of the allergen therein). On the other hand, when an allergen is used for the physiologically-active substance, then it is favorable for specific analysis of allergen, a cause of allergy (as to what type of allergic reaction occurs to what type of allergen and as to how the degree of allergic reaction is). When an anti-IgE antibody is used for the physiologically-active substance, then it is useful for determination of the overall IgE amount in a sample (as to whether or not the sample may cause allergic constitution to what degree of allergic reaction).

The analytical methods of the invention are described more concretely with reference to allergy-related analysis according to them.

### (1) Analysis of allergen:

First, a sample that contains a component to be analyzed (allergen) is applied to the physiologically-active substance-immobilized support in which the physiologically-active substance is an antibody to the allergen, in a mode of dropping application or coating, or the support is dipped in the sample, whereby the two are contacted with each other and a complex of the antibody and the allergen is thus formed. Next, the support is washed with a suitable buffer to thereby remove the components in the sample not having participated in the complex formation (e.g., free allergen). Next, the thus-formed complex is contacted with an antibody to the allergen that is labeled with a labeling substance (labeled binding substance having the property of specifically binding to the component or to the complex) in the same manner as above to thereby form a labeled complex of the immobilized antibody to the allergen, the allergen and the labeled antibody to the allergen. Next, this is washed with a suitable buffer to remove the free, labeled antibody not having participated in the formation of the labeled complex. Next, the labeling substance in the labeled complex is determined according to the method mentioned above, and it clarifies the presence or absence of the allergen in the sample.

Further, the amount of the labeling substance in the labeled complex in the above-mentioned method is determined according to the method mentioned above, and the thus-determined amount of the labeling substance is applied to the calibration curve that indicates the relationship between the allergen concentration, which is determined in the same manner as above by the use of other samples (standard samples) each having a known allergen concentration, and the actually-measured data (the amount of the labeling substance in the labeled complex actually measured herein). The process enables quantitative or semi-quantitative determination of the amount of the allergen existing in the sample.

In the above-mentioned method, when two or more different antibodies to two or more different allergens are immobilized on the carbon or metallic or semi-metallic carbon compound layer formed on the support and the thus-immobilized support is used, then multiple allergens of different types in one sample may be specified, identified and quantified in one operation, and this is an advantageous embodiment of the invention.

### (2) Specific analysis of allergen, cause of allergy:

First, a sample that contains a component to be analyzed (IgE to allergen) is applied to the physiologically-active substance-immobilized support in which the carbon or metallic or semi-metallic carbon compound layer formed on its surface has a physiologically-active substance, allergen (antigen) immobilized thereon, in a mode of dropping application or coating, or the support is dipped in the sample, whereby the two are contacted with each other and a complex of the allergen and IgE to the allergen is thus formed. Next, the support is washed with a suitable buffer to thereby remove the components in the sample not having participated in the complex formation (e.g., free IgE not intended for analysis). Next, the thus-formed complex is contacted with an anti-IgE antibody labeled with a labeling substance (labeled binding substance having the property of specifically binding to the component or to the complex) in the same manner as above to thereby form a labeled complex of the immobilized allergen, IgE to the allergen and the labeled anti-IgE antibody. Next, this is washed with a suitable buffer to remove the free, labeled anti-IgE antibody not having participated in the formation of the labeled complex. Next, the labeling substance in the labeled complex is determined according to the method mentioned above, and it clarifies the presence or absence of IgE to the specific allergen, the physiologically-active substance, in the sample, or that is, it clarifies as to whether or not the sample donor analyzed herein may have a cause of some allergic reaction to the specific allergen, and further in other words, it specifies the allergen, the cause of the allergy.

In addition, the amount of the labeling substance in the labeled complex in the above-mentioned method is determined according to the method mentioned above, and the thus-determined amount of the labeling substance is applied to the calibration curve that indicates the relationship between the IgE concentration, which is determined in the same manner as above by the use of other samples (standard samples) each having a known IgE concentration, and the actually-measured data (the amount of the labeling substance in the labeled complex actually measured herein). The process enables quantitative or semi-quantitative determination of the amount of the antibody (IgE) to the allergen, the physiologically-active substance existing in the sample. This shows the intensity of the susceptibility of the sample donor to the specific allergen, or that is, the degree of allergic reaction to the specific allergen that may occur in the sample donor.

The outline of the above-mentioned method is shown in Fig. 1.

In the above-mentioned method, when two or more different allergens are immobilized on the carbon or metallic or semi-metallic carbon compound layer formed on the support and the thus-immobilized support is used, then it may specify and identify the type of each allergen with which each IgE in the sample reacts (or that is, it clarifies as to whether or not the sample donor may have allergic reaction to what type of allergen), and this shows the intensity of the susceptibility of the sample donor to the thus-specified or identified allergen, and is therefore an advantageous embodiment of the invention.

### (3) Determination of overall IgE amount in sample:

First, a sample that contains a component to be analyzed (IgE) is applied to the physiologically-active substance-immobilized support in which the carbon or metallic or semi-metallic carbon compound layer formed on its surface has a physiologically-active substance, anti-IgE antibody immobilized thereon, in a mode of dropping application or coating, or the support is dipped in the sample, whereby the two are contacted with each other and a complex of the anti-IgE antibody and IgE is thus formed. Next, the support is washed with a suitable buffer to thereby remove the components in the sample not having participated in the complex formation. Next, the thus-formed complex is contacted with an anti-IgE antibody labeled with a labeling substance (labeled binding substance having the property of specifically binding to the component or to the complex) in the same manner as above to thereby form a labeled complex of the immobilized anti-IgE antibody, IgE and the labeled anti-IgE antibody. Next, this is washed with a suitable buffer to remove the free, labeled anti-IgE antibody not having participated in the formation of the labeled complex. Next, the amount of the labeling substance in the labeled complex is determined according to the method mentioned above. Thus determined, the amount of the labeling substance in the labeled complex is applied to the calibration curve that indicates the relationship between the IgE concentration, which is determined in the same manner as above by the use of other samples (standard samples) each having a known IgE concentration, and the actually-measured data (the amount of the labeling substance in the labeled complex actually measured herein), and this enables quantitative or semi-quantitative determination of the overall IgE amount in the sample. This shows the intensity of the susceptibility of the sample donor to general allergens, or that is, this shows as to whether or not the sample donor has an allergic constitution (concretely showing the degree of the allergic constitution of the sample donor).

The outline of the above-mentioned method is shown in Fig. 2.

In the above, when an anti-IgE antibody and two or more different allergens are all immobilized on the carbon or metallic or semi-metallic carbon compound layer formed on the support and the thus-immobilized support is used in carrying out both the two methods (2) and (3) at the same time, then it may specify and identify the type of the allergen to IgE in the sample (or that is, as to how the sample donor may have an allergic reaction to what allergen), and/or it may clarify the degree of the susceptibility of the sample donor to the thus-specified and identified allergen, further showing the degree of the susceptibility of the sample donor to general allergens (as to whether or not the sample donor has an allergic constitution), and is therefore an advantageous embodiment of the invention.

Concretely, a sample that contains a component to be analyzed (IgE) is applied to the physiologically-active substance-immobilized support in which the carbon or metallic or semi-metallic carbon compound layer formed on its surface has physiologically-active substances, anti-IgE antibody and two or more different allergens all on the same surface thereof, in a mode of dropping application or coating, or the support is dipped in the sample, whereby the two are contacted with each other and complexes of different allergens and IgE and a complex of the anti-IgE antibody and IgE are all formed. Next, the support is washed with a suitable buffer to thereby remove the components in the sample not having participated in the.complex formation. Next, the thus-formed complexes are contacted with an anti-IgE antibody labeled with a labeling substance in the same manner as above to thereby form labeled complexes of every immobilized allergen, IgE and the labeled anti-IgE antibody, and a labeled complex of the immobilized anti-IgE antibody, IgE and the labeled anti-IgE antibody. Next, this is washed with a suitable buffer to remove the free, labeled anti-IgE antibody not having participated in the formation of the labeled complexes. Next, of the thus-formed labeled complexes, the labeling substance in each immobilized allergen-related, labeled complex is determined according to the above-mentioned method, and this shows the presence or absence of an antibody (IgE) to which of two or more immobilized allergens in the sample, or that is, this specifies and identifies the type of the allergen to IgE in the sample. Further in other words, this clarifies as to whether the sample donor may have an allergic reaction to what allergy. In addition, when the amount of the antibody (IgE) to the thus-specified and identified allergen in the sample is quantitatively or semi-quantitatively determined according to the above-mentioned method on the basis of the amount of the labeling substance determined according to the method also mentioned above, then it clarifies the degree of the susceptibility of the sample donor to the thus-specified and identified allergen, or that is, it clarifies the degree of the allergic reaction which the sample donor may have to the allergen specified and identified herein. Further, when the overall IgE amount in the sample is quantitatively or semi-quantitatively determined according to the above-mentioned method on the basis of the amount of the labeling substance determined according to the method also mentioned above, then it clarifies the degree of the susceptibility of the sample donor to general allergens, or that is, it clarifies as to whether or not the sample donor has an allergic constitution (concretely showing the degree of the allergic constitution of the sample donor).

Next described is the kit of claim 22 of the invention, which is for analyzing a component in a sample.

The kit of claim 22 of the invention contains the physiologically-active substance-immobilized support of any of claims 1 to 12. On the support in the kit, immobilized are one or more physiologically-active substances capable of specifically binding to the corresponding one or more components to be analyzed with the kit.

In the kit of the invention, the preferred embodiments and the specific examples of the physiologically-active substance-immobilized support are the same as those mentioned hereinabove. Apart from the support therein, the kit of the invention may contain a labeled binding substance that has the property of specifically binding to the intended component or to a complex of the component.

The kit of the invention contains the physiologically-active substance-immobilized support of any of claims 1 to 12, and apart from it, the kit may contain any reagents that are generally used in the art. For example, it may contain a solution such as buffer (washing liquid),and a coloring agent such as oxidizable coloring reagent and coupling agent; and when the labeling substance in it is enzyme, it may contain a substrate for the enzyme, a reagent such as reaction stopper for stopping the enzymatic reaction; and it may contain a standard product of the component to be analyzed with the kit. The concentration of each reagent and each standard product that may be in the kid may be any ordinary one generally employed in the art.

### EXAMPLES

The invention is described with reference to the following Examples.

### (Example 1)

According to the process of Fig. 1, IgE was analyzed by the use of an anti-IgE antibody-bound, diamond-coated support.

### 1. Activation of functional group:

A carboxyl group-coated, diamond-coated support was dipped in a 0.1 M phosphate buffer (pH 6) with 20 mM N-hydroxysuccinimide and 0.1 M 1-(3-(dimethylamino)propyl)-3-ethylcarbodiimide therein, for 30 minutes. After thus dipped, the diamond-coated support was washed twice with germ-free distilled water, and centrifuged to completely remove water from it.

### 2. Immobilization of anti-human IgE antibody:

An anti-human IgE antibody was processed with 50 mM MOPS buffer (pH 7.5) to have a predetermined concentration, and spotted onto the activated, diamond-coated support that had been prepared in the above, by the use of a stamping machine, Nippon Laser Electronics' GT-MASS.

After thus spotted, this was incubated in a 50 % formamide-containing, humidified chamber that had been previously controlled at 65°C, for 1 hour.

### 3. Blocking:

The IgE antibody-immobilized, diamond-coated support that had been prepared in the above 2 was blocked with 2 % bovine serum albumin-containing 50 mM MOPS buffer (pH 7.5) for one full day at room temperature. After thus blocked, this was centrifuged at 1000 rpm for 1 minute.

### 4. Reaction with specimen:

A component to be analyzed, human IgE was processed with 2 % bovine serum albumin-containing 50 mM MOPS buffer (pH 7.5) to prepare specimens of predetermined concentrations. 10 µl of each specimen was applied to a chip of the support, covered with a glass cover so as to protect it from air bubbles, then put into a hybridization chamber and reacted at room temperature for 1 hour. After the reaction, the glass cover was removed from it, and each chip was washed three times with SSC (0.15 M NaCl-0.015 M trisodium citrate solution), and centrifuged at 1000 rpm for 1 minute.

### 5. Detection of human IgE bound to the support:

10 µl of Cy3-labeled anti-human IgE antibody (0.86 µmg/ml) was applied to the chip, covered with a glass cover so as to protect it from air bubbles, then put into a hybridization solution and reacted at room temperature for 1 hour. After the reaction, the glass cover was removed from it, and each chip was washed five times with SSC (0.15 M NaCl-0.015 M trisodium citrate solution), and centrifuged at 1000 rpm for 1 minute.

Using a confocal laser scanner by GSI Lumonicus, the amount of the Cy3-labeled anti-human-IgE antibody was determined, and this indicates the degree of antigen-antibody reaction on each chip. The data are shown in Table 1 and in Fig. 3.

**Table 1 -**

| Data of Anti-human IgE Antibody Amount | | | | |
|---|---|---|---|---|
| | | IgE Concentration | | |
| | | 0 IU/ml | 0.7 IU/ml | 7 IU/ml |
| Immobilized Anti-human IgE Antibody Concentration | 0.1 mg/ml | 5335 | 9950 | 11035 |
| | 0.5 mg/ml | 13957 | 21119 | 22578 |
| | 1 mg/ml | 20233 | 39233 | 45382 |

From the data in Table 1 and Fig. 3, it is understood that the method of the invention enables the detection and the quantification of human IgE.

### (Example 2)

According to the process of Fig. 2, allergen was analyzed by the use of an anti-IgE antibody-bound, diamond-coated support.

### 1. Activation of diamond-coated support:

A carboxyl group-coated, diamond-coated support was dipped in a 0.1 M phosphate buffer (pH 6) with 20 mM N-hydroxysuccinimide and 0.1 M 1-(3-(dimethylamino)propyl)-3-ethylcarbodiimide therein, for 30 minutes. After thus dipped, this was washed twice with germ-free distilled water, and centrifuged to completely remove water from it.

### 2. Immobilization of allergen:

An allergen (mite antigen or Japanese cedar pollen antigen) was processed with 50 mM MOPS buffer (pH 7.5) to have a concentration of 0.5 mg/ml, and spotted onto the activated, diamond-coated support that had been prepared in the above, by the use of a stamping machine, Nippon Laser Electronics' GT-MASS. After thus spotted, this was incubated in a 50 % formamide-containing, humidified chamber for 1 hour.

### 3. Blocking:

10 µl of 2 % bovine serum albumin-containing 50 mM MOPS buffer (pH 7.5) was applied to the diamond-coated support, which was covered with a glass cover to protect it from air bubbles and was thus blocked for 2 hours.

The glass cover was removed from it, and the support was washed three times with 1 × SSC, and then centrifuged to remove the excess water.

### 4. Application of specimen:

A human serum positive to allergic reaction with Japanese cedar pollen or with mite (Japanese cedar pollen-positive serum or mite-positive serum), and a human serum negative to the two (negative serum) were prepared as specimens. 10 µl of each serum specimen was applied to a microarray of the support, covered with a glass cover so as to protect it from air bubbles, and reacted for one full day at 4°C. After the reaction, the glass cover was removed from it, and each microarray was washed three times with 1 × SSC, and centrifuged to remove the excess water.

### 5. Reaction with labeled antibody:

10 µl of Cy3-labeled anti-human IgE antibody (4.3 µg/ml) in 2 % bovine serum albumin-containing 50 mM MOPS buffer (pH 7.5) was applied to the microarray, covered with a glass cover so as to protect it from air bubbles, and reacted for 3 hours. After the reaction, the glass cover was removed from it, and each microarray was washed three times with 1 × SSC, and centrifuged to remove the excess water.

### 6. Result:

Using a confocal laser scanner by GSI Lumonicus, the Cy3-labeled anti-human-IgE antibody-derived fluorescence was determined, and this indicates the degree of antigen-antibody reaction on each microarray. The result of negative serum specimen is shown in Fig. 4; that of Japanese cedar pollen-positive serum specimens is in Fig. 5; and that of mite-positive serum specimens is in Fig. 6.

From the result in Fig. 4, it is understood that the negative serum specimen gave little Cy3-labeled anti-human IgE antibody-derived fluorescence both in the Japanese cedar pollen antigen immobilization site and in the mite immobilization site on the microarray. From the result in Fig. 5, it is understood that the Japanese cedar pollen-positive serum specimen gave clear Cy3-labeled anti-human IgE antibody-derived fluorescence in the Japanese cedar pollen antigen immobilization site on the microarray but little in the mite immobilization site thereon. From the result in Fig. 6, it is understood that the mite-positive serum specimen gave intensive Cy3-labeled anti-human IgE antibody-derived fluorescence in the mite antigen immobilization site on the microarray.

In Fig. 6, a little and weak Cy3-labeled anti-human IgE antibody-derived fluorescence is found in the Japanese cedar pollen antigen immobilization site on the microarray. In this respect, the mite-positive serum donor was examined in detail. As a result, it was found that the donor was positive not only to mite allergic reaction but also to Japanese cedar pollen allergic reaction in some degree, or that is, the mite-positive serum tested herein was positive to allergic reaction both with mite and with Japanese cedar pollen.

From these results, it is understood that the method of the invention makes it possible to specifically identify allergy-causing allergens and makes it possible to determine the degree of the susceptibility of specimen donors to the thus-identified allergens.

### INDUSTRIAL APPLICABILITY

The support of the invention may immobilize a physiologically-active substance at high density thereon, and the physiologically-active substance-immobilized support realizes a high immobilization efficiency not consuming a large amount of the substance. In addition, the support may ensure the high immobilization efficiency even for physiologically-active substances with various surface charges.

The method of the invention produces the support efficiently.

In the method of the invention for analyzing a component in a sample, the support is used, and the method enables accurate analysis of various components in a sample.

Further, using the kit of the invention, it is possible to analyze components in a sample in a more simplified manner.

In particular, since the support with an anti-IgE antibody immobilized thereon can react with allergen to accurately analyze IgE that has a close correlation to allergy, it is useful for various immunodiagnoses for determining the cause of allergy.

Accordingly, the invention is useful in various fields, for example, for disease diagnoses in the medical field, and for studies in the biochemical and molecular biology field.

## Claims

1. A physiologically-active substance-immobilized support which has a carbon or metallic or semi-metallic carbon compound layer formed on its surface and has a physiologically-active substance immobilized on the layer.

2. The physiologically-active substance-immobilized support as claimed in claim 1, wherein the physiologically-active substance is immobilized on the support surface via the functional group of the carbon or metallic or semi-metallic carbon compound layer.

3. The physiologically-active substance-immobilized support as claimed in claim 1, wherein the physiologically-active substance is immobilized on the support surface through binding of the functional group of the substance to the functional group of the carbon or metallic or semi-metallic carbon compound layer.

4. The physiologically-active substance-immobilized support as claimed in claim 3, wherein the functional group of the physiologically-active substance and/or the functional group of the carbon or metallic or semi-metallic carbon compound layer is one or more selected from a group consisting of a hydroxyl group, a carboxyl group, a sulfo group, a cyano group, a nitro group, a thiol group, an amino group, an aminophenyl group and an epoxy group.

5. The physiologically-active substance-immobilized support as claimed in any of claims 1 to 4, wherein the physiologically-active substance is a substance that has a specific action on organisms or a biological protein or peptide.

6. The physiologically-active substance-immobilized support as claimed in any of claims 1 to 5, wherein the physiologically-active substance is an antigen and/or an antibody.

7. The physiologically-active substance-immobilized support as claimed in claim 6, wherein the antigen and/or antibody is one or more selected from a group consisting of tumor markers, hormones, hormone-disturbing chemicals, microorganisms-derived proteins or peptides or sugar chain antigens, receptors, ligands, allergens, immunoglobulins, lectins, sugar chains, lipids, lipopolysaccharides and antibodies to these.

8. The physiologically-active substance-immobilized support as claimed in claim 7, wherein the antigen and/or antibody is one or more selected from a group consisting of allergens, immunoglobulins and antibodies to these.

9. The physiologically-active substance-immobilized support as claimed in any of claims 1 to 8, wherein the carbon or metallic or semi-metallic carbon compound layer comprises crystalline carbon.

10. The physiologically-active substance-immobilized support as claimed in any of claims 1 to 8, wherein the carbon or metallic or semi-metallic carbon compound layer comprises amorphous carbon.

11. The physiologically-active substance-immobilized support as claimed in claim 9, wherein the crystalline carbon is diamond or diamond-like carbon.

12. The physiologically-active substance-immobilized support as claimed in claim 10, wherein the amorphous carbon is graphite or amorphous carbon.

13. A method for producing a physiologically-active substance-immobilized support, which comprises contacting a physiologically-active substance with a support having a carbon or metallic or semi-metallic carbon compound layer with a functional formed on its surface.

14. A method for producing a physiologically-active substance-immobilized support, which comprises introducing a functional group into a support surface having a carbon or metallic or semi-metallic carbon compound layer formed thereon, then contacting it with a physiologically-active substance.

15. The method for producing a physiologically-active substance-immobilized support as claimed in claim 13 or 14, wherein the functional group is one or more selected from a group consisting of a hydroxyl group, a carboxyl group, a sulfo group, a cyano group, a nitro group, a thiol group, an amino group, an aminophenyl group and an epoxy group.

16. A physiologically-active substance immobilized on the surface of a support having a carbon or metallic or semi-metallic carbon compound layer formed thereon.

17. A method for analyzing a component in a sample, in which is used the physiologically-active substance-immobilized support of any of claims 1 to 12.

18. The method for analyzing a component in a sample as claimed in claim 17, wherein the physiologically-active substance-immobilized support of any of claims 1 to 12 is contacted with a sample that contains the component to be analyzed and the resulting complex of the physiologically-active substance and the component is analyzed.

19. The method for analyzing a component in a sample as claimed in claim 17 or 18, wherein the physiologically-active substance immobilized on the support surface is any of antigen or antibody and the component in the sample is an antigen or antibody to the physiologically-active substance.

20. The method for analyzing a component in a sample as claimed in claim 19, wherein the antigen or antibody is one or more selected from a group consisting of tumor markers, hormones, hormone-disturbing chemicals, microorganisms-derived proteins or peptides or sugar chain antigens, receptors, ligands, allergens, immunoglobulins, lectins, sugar chains, lipids, lipopolysaccharides and antibodies to these.

21. The method for analyzing a component in a sample as claimed in claim 20, wherein the antigen or antibody is more preferably one or more selected from a group consisting of allergens, immunoglobulins and antibodies to these.

22. A kit for analyzing a component in a sample, which contains the physiologically-active substance-immobilized support of any of claims 1 to 12.
